# EUROPÄISCHE PATENTSCHRIFT

(11) **EP 0 444 561 B1**
(45) Veröffentlichungstag und Bekanntmachung des Hinweises auf die Patenterteilung: **20.11.1996**
(21) Anmeldenummer: 91102712.6
(22) Anmeldetag: 25.02.1991
(51) Int. Cl.: G01N 33/53, G01N 33/543, G01N 33/563, G01N 33/58, C12Q 1/68

(54) **Verfahren zur immunologischen Bestimmung von Liganden**
Method for the immunological determination of ligands
Méthode pour la détermination immunologique de ligands

(30) Priorität: 26.02.1990 DE 4006054
(43) Veröffentlichungstag der Anmeldung: 04.09.1991
(73) Patentinhaber: BOEHRINGER MANNHEIM GMBH, 68298 Mannheim (DE)
(72) Erfinder: Deger, Arno, Dr., W-8124 Seeshaupt (DE); Schenk, Roland, Dr., W-8120 Weilheim (DE); Bienhaus, Gerhard, Dr., W-8121 Haunshofen (DE)
(74) Vertreter: Huber, Bernhard, Dipl.-Chem.

(56) Entgegenhaltungen:
- EP-A- 0 019 277
- EP-A- 0 160 900
- EP-A- 0 344 578
- EP-A- 0 363 942
- US-A- 4 228 237

## Beschreibung

Die Erfindung betrifft ein immunologisches Verfahren zur Bestimmung eines Liganden sowie eine dazu geeignete Reagenzien-Zusammensetzung.

In Körperflüssigkeiten und Geweben kommen sehr viele Substanzen vor, die mit einem spezifischen Bindungspartner bindefähig sind und als Parameter für bestimmte Erkrankungen oder den Gesundheitszustand des menschlichen Körpers dienen. Hierzu zählen einerseits immunologisch aktive Proteine, die Bindungsstellen an ihrer Oberfläche aufweisen wie z.B. Tumormarker, Hormone oder virale Proteine und andererseits DNA-Fragmente. Da diese nachfolgend als "Ligand" bezeichneten Substanzen oft nur in sehr geringen Mengen vorkommen, verwendet man zu ihrem Nachweis Verfahren nach dem Prinzip des Immunoassays, mit denen diese Substanzen sehr spezifisch und genau bestimmt werden können. Die bekannten immunologischen Bestimmungsverfahren lassen sich in homogene und heterogene Verfahren einteilen. Bei den heterogenen Verfahren ist immer eine Festphasen-Reaktion beteiligt, um Komplexe, die die nachzuweisende Substanz und eine markierte Komponente enthalten, zu immobilisieren und dadurch von ungebundenen Komponenten abzutrennen. Bei den homogenen Verfahren erfolgt keine Trennung von gebundener Markierung und ungebundener Markierung, so daß eine Differenzierung von gebundener und ungebundener Markierung durch andere Methoden erfolgen muß.

Die heterogenen Immunoassays basieren im wesentlichen auf zwei Varianten, nämlich kompetitiven und Sandwich-Assays. Bei diesen Verfahrensvarianten werden in der Regel mindestens zwei Rezeptoren eingesetzt, die mit dem nachzuweisenden Liganden bindefähig sind und von denen einer eine Markierung trägt und der andere an eine Festphase gebunden ist oder die Bindung an eine feste Phase vermittelt. Eine Vielzahl von Varianten sind hierzu bekannt unter Verwendung weiterer Rezeptoren. In die Bestimmung gehen nun alle Komplexe ein, die an der Festphase gebunden sind und eine Markierung aufweisen.

Zur Durchführung von kompetitiven heterogenen Immunoassays gibt es im wesentlichen zwei Varianten, wobei entweder ein Antikörper gegen den Liganden immobilisiert wird oder eine zum Liganden analoge Substanz immobilisiert wird. In der ersten Variante wird eine Probelösung, die den Liganden enthält und ein Konjugat aus Ligand und einer Markierung mit dem immobilisierten Antikörper inkubiert. Dabei konkurrieren Ligand und markierte Substanz um die Bindung an den Antikörper. Je mehr Ligand in der Lösung vorhanden ist, desto weniger markierte Substanz kann gebunden werden. Nach der Trennung von fester und flüssiger Phase kann dann die Markierung in einer der beiden Phasen bestimmt werden. Die Menge an gebundener markierter Substanz ist ein indirektes Maß für die Menge an zu bestimmender Substanz, also an Ligand.

In der zweiten Variante wird die Probelösung, die den Ligand enthält, mit einem für sie spezifischen Antikörper sowie dem immobilisierten Substanz-Analogen inkubiert. Hierbei konkurrieren immobilisierter Ligand und in der Lösung vorhandener Ligand um die Bindung an den Antikörper. Je mehr Ligand in der Lösung vorhanden ist, desto weniger Antikörper wird durch Bindung an den immobilisierten Ligand analog an die Festphase gebunden. Auch hier wird wiederum nach Trennung der festen Phase von der flüssigen Phase die Menge an gebundener Markierung bestimmt, die wieder indirekt proportional zur Menge an Ligand in der Probelösung ist.

Bei den Varianten des Sandwich-Immunoassays wird ein Antikörper gegen den Ligand immobilisiert und die Probelösung in Gegenwart dieses immobilisierten Antikörpers mit einem weiteren Antikörper, der mit dem Ligand bindefähig ist und markiert ist, inkubiert. Die Rezeptoren werden dabei im Überschuß zugegeben, so daß alle in der Probe befindlichen nachzuweisenden Moleküle an der Festphase gebunden werden und mit einem markierten Rezeptor binden.

Ein Nachteil dieser Verfahren ist es, daß für jede zu bestimmende Substanz (Ligand) mehrere speziell angepaßte Rezeptoren vorgesehen werden müssen. So sind häufig markierte spezielle Antikörper erforderlich, die von Ligand zu Ligand verschieden sind. Die kovalente Bindung von Markierungsgruppen an Antikörper, die üblicherweise an mehreren Stellen des Antikörpermoleküls angreift, kann unerwünschte Veränderungen der Bindeeigenschaften des so modifizierten Antikörpers hervorrufen.

EP-A-0 344 578 offenbart ein Verfahren wobei eine avidin-beschichtete Festphase für die Bindung von biotinylierten, ligandspezifischen Antikörpern und enzym-markierte ligandspezifische Antikörper für die Detektion verwendet werden.

US-A-4,228,237 beschreibt Verfahren zur immunologischen Bestimmung, die enzym-markiertes Avidin und biotinylierte spezifische Antikörper für die Detektion und eine Festphase mit spezifischen Antikörper verwenden.

Ein weiteres Problem tritt auf beim Nachweis von Proteinen oder DNA- oder RNA-Fragmenten, die mehr als eine Bindungsstelle aufweisen.

Hier kann jeweils eine Mehrzahl oder Vielzahl von Rezeptoren binden, wobei die daraus resultierende Ungenauigkeit durch Einsatz von Standards noch zu kompensieren ist. Diese Ungenauigkeit wird jedoch noch potenziert, wenn alle eingesetzten Rezeptoren selbst wieder bi- oder polyvalent sind.

Es war daher Aufgabe der Erfindung, ein Verfahren zur Verfügung zu stellen, mit dem Liganden mit hoher Genauigkeit, guter Reproduzierbarkeit und hoher Spezifität nachgewiesen werden können und bei dem außerdem universell einsetzbare, markierte Komponenten und universell geeignete Festphasenmaterialien verwendet werden können. Insbesondere soll nur ein einziger speziell angepaßter Rezeptor erforderlich sein.

Diese Aufgabe wird gelöst durch ein Verfahren zur immunologischen Bestimmung eines Liganden, welches dadurch gekennzeichnet ist, daß man den zu bestimmenden Liganden mit
a) mindestens 2 Molekülen einer für P1 spezifischen bindefähigen Substanz P2, von denen mindestens eines dieser Moleküle eine Markierung trägt, und
b) einem Reagenz, umfassend Rezeptoren R, die den Liganden spezifisch binden, wobei jeder Rezeptor aus einem einzigen Molekül eines für P2 monovalent bindefähigen Bindungspartners P1 und dem einzig spezifischen Bindungsmolekül R₁ für den zu bestimmenden Liganden besteht und wobei P1 in allen Rezeptoren R gleich ist und R₁ in allen Rezeptoren gleich sein kann, umsetzt und dann die Markierung bestimmt.

Gemäß Merkmal a) wird markiertes P2 in bekannter Menge bevorzugt im Überschuß bezüglich der möglichen Menge an Ligand eingesetzt, wobei dieser Überschuß, wenn alles P2 markiert ist, auf die doppelte stöchiometrische Menge an Ligand bezogen ist, wenn auch unmarkiertes P2 eingesetzt wird, auf die einfach stöchiometrische Menge Ligand bezogen ist. Bei der letzteren Ausführungsform liegt auch unmarkiertes P2 im einfachen Überschuß vor.

Das erfindungsgemäße Verfahren ist geeignet zur Bestimmung aller in Körperflüssigkeiten oder Gewebe-Extrakten nachzuweisenden, zu einer spezifischen Bindung befähigten Liganden, wobei niedrig konzentrierte Substanzen ebenso gut nachweisbar sind wie hoch konzentrierte. Die Empfindlichkeit und Genauigkeit des Verfahrens ist gegenüber den bisher bekannten Verfahren verbessert. Die Erfindung bietet die Möglichkeit, mit einfachen Reagenzien schnell und zuverlässig Bestimmungen durchzuführen.

Das Verfahren ist besonders geeignet zur Bestimmung von Liganden mit mehreren Bindungsstellen und von DNA- oder RNA-Molekülen. Unter spezifischer Bindungsstelle wird dabei eine Bindungsstelle verstanden, die eine spezifische Bindung mit einer anderen Substanz eingehen kann. Beispiele hierfür sind antigene Determinanten, spezifische Bindungsstellen auf Proteinen oder eine spezifische Nukleinsäuresequenz auf DNA oder RNA.

Wesentliches Merkmal ist, daß jeder Rezeptor R einen einzigen Partner P1 des spezifisch bindenden Paares P1/P2 enthält und dieser Partner P1 gegenüber P2 monovalent ist, so daß nur ein einziges Molekül P2 pro Molekül Rezeptor R gebunden werden kann. Als Komponente R₁ sind Substanzen geeignet, die mit dem Liganden spezifisch bindefähig sind. Dies können z.B. Makromoleküle wie Antikörper, Antikörper-Fragmente und spezifisch bindefähige Bindeproteine, Haptene, Epitope oder, im Falle eines DNA- oder RNA-Nachweises, DNA-Sonden sein. Bevorzugt weist die Komponente R₁ nur eine Bindungsstelle für den Liganden auf. Im Falle eines Proteinnachweises werden bevorzugt Antikörper-Fragmente verwendet, die nur ein Paratop haben oder Substanzen, mit denen das Protein spezifisch eine Bindung eingeht, beispielsweise T4 zum Nachweis von T4 bindendem Protein. Für den Nachweis von DNA oder RNA wird als Komponente R₁ z.B. eine Sonde verwendet, die mit einer Sequenz der nachzuweisenden DNA oder RNA hybridisieren kann. Besonders bevorzugt wird als Komponente R₁ im Rezeptor R ein Fab'-Fragment eines mit dem Ligand bindefähigen Antikörpers verwendet. R₁ kann abhängig vom Liganden eine einheitliche Substanz oder eine Mischung sein.

Die Komponente P1 des Rezeptors R ist ein Partner eines spezifisch bindenden Paares. Spezifisch miteinander bindende Paare sind bekannt. Geeignete Bindungspaare sind insbesondere Biotin-Streptavidin bzw. Avidin; Hapten-Antikörper, Antigen-Antikörper, Concavalin-Antikörper; Zucker-Lectin; Hapten-Bindeprotein, z.B. Thyroxin bindendes Globulin und Thyroxin- oder Oligopeptid-Antikörper.

Besonders bevorzugt wird als bindendes Paar Biotin und Streptavidin bzw. Avidin eingesetzt, so daß Rezeptor R₁ besonders bevorzugt ein einziges Molekül Biotin als Partner P1 enthält.

Die Herstellung der Konjugate erfolgt unter Anwendung der dem Fachmann bekannten Methoden (z.B. analog Eur. J. Biochem. 131 (1980) 333-338).

Im Falle einer heterogenen Verfahrensführung vermittelt P2 in nicht markierter Form die Bindung an die feste Phase.

Erfindungsgemäß wird erreicht, einen Universaltest zur Verfügung zu stellen, da feste Phase und Markierungskonjugat für alle Bestimmungen gleichbleiben können und nur ein einziger Rezeptor auf den jeweils nachzuweisenden Liganden abgestimmt werden muß. Da die Bindung von R, Ligand und markiertem P2 in homogener Phase abläuft, ist sie gegenüber der Bindung von R an die Festphasen P2, die in heterogener Phase abläuft, bevorzugt. Das an die Festphase gebundene nicht markierte P2 des spezifisch bindenden Systems wird daher im Überschuß verwendet.

Bevorzugt werden als Rezeptor R vorzugsweise Konjugate verwendet, die aus einem Antikörper oder Antikörper-Fragment R₁ und Biotin als P1 bestehen.

Die Herstellung der Konjugate R erfolgt in an sich bekannter Weise durch Umsetzung der Komponente R₁, z.B. eines Antikörpers bzw. Antikörperderivats mit dem Partner P1 im stöchiometrischen Verhältnis von 1:1. Vorzugsweise erfolgt die Kopplung über eine freie SH-Gruppe oder eine Aminogruppe. Die dabei statistisch entstehen-den Produkte mit zwei oder mehr Komponenten P1 können durch gelchromatographische Verfahren abgetrennt werden. Besonders bevorzugt werden für das erfindungsgemäße Verfahren Konjugate R eingesetzt, die aus einem Fab'-Fragment und Biotin bestehen. Zur Herstellung derartiger Konjugate wird z.B. ein mit dem nachzuweisenden Liganden bindefähiger Antikörper mit Pepsin behandelt, das entstehende F(ab')₂-Fragment reduzierenden Bedingungen ausgesetzt und anschließend mit der Komponente P1 umgesetzt, wobei entweder über eine in der Komponente P1 vorhandene funktionelle Gruppe oder über einen Spacer gegebenenfalls nach Aktivierung der Bindungsstellen die Komponente P1 an einer freien SH-Gruppe oder Aminogruppe des Fab'-Fragmentes bindet. Produkte, die einen oder mehrere Partner P1 enthalten, können beispielsweise nach einem in EP-A 0 131 343 beschriebenen Verfahren aufgetrennt werden, wobei die Ladungsdifferenz verwandter Proteine zur Auftrennung herangezogen wird. Bei Bindung des Partners über die Amino-Funktionen eines Proteins führt Einfach- oder Mehrfachbindung zu Konjugaten mit unterschiedlicher Ladung, was ihre Trennung ermöglicht.

Als feste Phase besonders geeignet sind Reagenzgläschen oder Mikrotiterplatten aus Polystyrol und ähnlichen Kunststoffen, die adsorptiv oder kovalent an der Innenoberfläche mit P2 beschichtet sind. Weiterhin geeignet sind auch teilchenförmige Substanzen, z.B. Molekularsiebmaterialien, Glasperlen, Kunststoffschläuche und dergleichen sowie poröse schichtförmige Träger wie Papier. Die Bindung des Partners P2 erfolgt in an sich bekannter Weise.

Mindestens ein Teil von P2, bei homogener Testführung alles P2, wird in markierter Form eingesetzt. Als Markierung eignen sich dabei die üblichen Markierungen immunologischer Tests, z.B. ein Enzym, eine fluoreszierende, chemilumineszierende oder radioaktive Substanz. Verfahren zur Markierung sind dem Fachmann bekannt, z.B. aus Clin.Chim. Acta 81 (1977) 1-40 und bedürfen hier keiner weiteren Erläuterung. Die Markierung kann in an sich bekannter Weise bestimmt werden.

Wird die Erfindung mit homogener Verfahrensführung eingesetzt, d.h. ohne Verwendung einer festen Phase, so wird alles P2 in markierter Form verwendet. Die dabei gebildeten Komplexe aus Ligand, mindestens zwei Molekülen R und einer der Anzahl R im Komplex entsprechenden Anzahl von Molekülen P2 markiert wird dann nach den dem Fachmann bekannten Methoden zur Messung einer Markierung bei homogener Verfahrensführung nachgewiesen. Geeignete Nachweismethoden sind beispielsweise beschrieben in EP-B 0084 807.

Sowohl bei heterogener als auch bei homogener Führung des erfindungsgemäßen Verfahrens kann zusätzlich ein Konjugat K eingesetzt werden, welches den Ligand oder ein Ligand-Analog mit einem Bindepartner P1 enthält, der mit dem Bindepartner P1 im Rezeptor R identisch ist. Bei dieser Verfahrensführung konkurrieren Ligand in der Probelösung mit Ligand oder Ligand-Analog im zugesetzten Konjugat K um die Komponente R₁ im Rezeptor R, so daß sich ein kompetitives Verfahren ergibt. Diese Ausführungsform kann sowohl beim Festphasenverfahren als auch beim Verfahren in homogener Phase angewendet werden. Für die Herstellung des Konjugats K gelten die Ausführungen zur Herstellung des Rezeptors R in gleicher Weise. Als Ligand-Analog wird dabei eine Substanz angesehen, die hinsichtlich ihrer Bindungsfähigkeit mit der Komponente R₁ im Rezeptor R ähnliche Eigenschaften wie der Ligand selbst aufweist, so daß tatsächlich eine Konkurrenz um die Bindungsstelle unter den eingesetzten stöchiometrischen Bedingungen vorliegt.

Das Verfahren kann in einer oder mehr Stufen durchgeführt werden. Die Auswertung erfolgt in an sich bekannter Weise. Da jeder der Rezeptoren und auch die zu bestimmende Substanz jeweils nur spezifisch mit dem für ihn bestimmten Reaktionspartner reagieren kann, ist es möglich, alle Rezeptoren und die Probe zusammen zu inkubieren und das Verfahren einstufig durchzuführen. Dies ist besonders vorteilhaft bei der Durchführung des Verfahrens in einem Analyseautomaten. Für den Fall, daß das Verfahren einstufig heterogen durchgeführt wird, wird vorzugsweise die Substanz P2, die eine Markierung trägt, im Unterschuß gegen über dem Rezeptor R eingesetzt.

Die Durchführung aller Verfahrensvarianten erfolgt vorzugsweise in einer gepufferten Lösung. Puffersysteme für diese Verfahren sind an sich bekannt. Besonders geeignet sind hierfür GOOD-Puffer und Phosphatpuffer.

Zur Durchführung des erfindungsgemäßen Verfahrens in heterogener Phase wird die Probelösung mit Rezeptoren R, markiertem P2 und gegebenenfalls K in Gegenwart einer mit P2 unmarkiert beschichteten Festphase entweder gleichzeitig oder nacheinander inkubiert. Dabei binden beispielsweise zwei Rezeptoren R an den Liganden über die Komponenten R₁. Über Partner P1 des einen Rezeptors R wird dann markierter P2 gebunden, und über den anderen Rezeptor R erfolgt durch das Bindepaar P1/P2 unmarkiert die Bindung an die feste Phase. In die Auswertung gehen alle Komplexe ein, die an der Festphase gebunden sind und eine Markierung tragen.

Erfindungsgemäß wird ein Verfahren zur Verfügung gestellt, das einfach und schnell durchzuführen ist und auch bei Verwendung polyklonaler Antikörper sehr empfindlich ist.

Ein weiterer Gegenstand der vorliegenden Erfindung ist ein Reagenz zur immunologischen Bestimmung eines Liganden, welches dadurch gekennzeichnet ist, daß es
a) mindestens 2 Moleküle einer für P1 spezifisch bindefähigen Substanz P2, von denen mindestens eines dieser Moleküle eine Markierung trägt, und mindestens ein weiteres Molekül P2 an eine Festphase gebunden vorliegt oder alles P2 eine Markierung trägt und keine Festphase vorliegt.
b) ein Reagenz, umfassend Rezeptoren R, die den Liganden spezifisch binden, wobei jeder Rezeptor aus einem einzigen Molekül eines für P2 monovalent bindefähigen Bindungspartners P1 und dem einzig spezifischen Bindungsmolekül R₁ für den zu bestimmenden Liganden besteht und wobei P1 in allen Rezeptoren R gleich ist und R₁ in allen Rezeptoren gleich sein kann, enthält.
Vorzugsweise enthält das Reagenz die Substanz P2 in markierter Form und als festphasengebundene Form in vereinigter Konfektionierung (z.B. Schicht im Testträgerdevice oder magm. Partikellösung).

Gemäß einer bevorzugten Ausführungsform enthält dieses Reagenz unmarkiertes, an eine Festphase gebundenes oder mit mit einer Festphase bindefähiges P2. Gemäß einer weiteren bevorzugten Ausführungsform enthält das Reagenz zusätzllich ein Konjugat K aus Ligand oder Ligand-Analog und einer Komponente P1 wie vorstehend definiert.

Dieses Reagenz ist zur Bestimmung einer Vielzahl von Parametern in Körperflüssigkeiten und Gewebeextrakten geeignet.

In einer bevorzugten Ausführungsform enthält das Reagenz zusätzlich Puffersubstanzen. Besonders bevorzugt enthält es Phosphatpuffer oder GOOD-Puffer.
- Fig. 1: zeigt ein Diagramm, in dem die Ergebnisse für die Bestimmung des Thyroxin-Bindungsindex aufgetragen sind.
- Fig. 2: zeigt ein Diagramm, in dem die Ergebnisse für die Bestimmung von Anti-T4-Antikörper aufgetragen sind.

Die Erfindung wird durch die Figur und die Beispiele erläutert.

### Beispiel 1

### Bestimmung von AFP (α-Föto-Protein)

a) Herstellung eines Konjugats aus Biotin und Fab'-Fragmenten von Anti-AFP-Antikörpern (Anti-AFP-Fab'-Biotin)
   Polyklonale Antikörper gegen AFP werden immunsorptiv auf gereinigt und hieraus Fab'-Fragmente hergestellt. Diese werden nach Analyt.Biochem. 161 (1987) 262-271 bzw. Analyt.Biochem. 149 (1985) 529-536 an Biotin gekoppelt.
b) Testdurchführung
   Puffer A:
   120 mmol/l Natriumbarbiturat
   18,2 mmol/l Phosphatpuffer, pH 8,6
   1,27 mmol/l 8-Anilino-1-naphtalinsulfonsäure
   0,2 Gew.-% Rinderserumalbumin
   (Endkonzentration im Test)

480 µl Puffer A und 20 µl Anti-AFP-Fab'-Biotin (Endkonzentration im Test: 4 µg/ml) werden in ein mit Streptavidin beschichtetes Polystyrolgefäß (Herstellung nach EP-A 0 269 092) zusammen mit 50 µl Probe (Humanserum aufgestockt mit AFP) gegeben und 30 Minuten bei 25°C inkubiert. Anschließend werden 480 µl Puffer A und 20 µl einer Lösung eines Streptavidin-POD-Konjugats (50 mU/ Test) zugegeben und 30 Minuten bei 25°C inkubiert. Es wird gewaschen und 1 ml ABTS®-Lösung (9,1 mmol/l ABTS®, 2,2'-Azino-di[3-ethylbenzthiazolin-sulfonsäure(6)]-di-ammoniumsalz, 100 mmol/l Phosphat-Citrat-Puffer, pH 4,4, 3,2 mmol/l Natriumperborat) zugegeben, bei 25°C 30 Minuten inkubiert und die optische Dichte bei 422 nm als Maß für den AFP-Gehalt bestimmt.

### Beispiel 2

### T-Uptake-Test

Die Bestimmung erfolgt in der Weise, daß T4 zur Probe zugegeben wird, um überschüssiges TBG abzusättigen. Danach wird das nicht gebundene T4 gemessen. Damit wird eine dem Thyroxinbindungsindex (TBI) direkt proportionale Eichkurve erhalten.

Probe: Als Proben werden Standards verwendet, die in Humanserum definierte Mengen TBG und T4 enthalten. Für Probe 1 ergibt sich ein TBI von 0,44 und für Probe 2 ein TBI von 1,44 (vgl. Arbeitsanleitung zu Enzymun-Test® TBK von Boehringer Mannheim GmbH, Bestell-Nr. 249416).

### Reagenz 1

100 U/l Konjugat aus Streptavidin und POD
36 pmol/l T4
120 mmol/l Natriumbarbiturat
18,2 mmol/l Phosphatpuffer, pH 8,6
0,2 Gew.-% Rinderserumalbumin

### Reagenz 2

0,2 nmol/l Konjugat aus T4 und Biotin
0,15 mg/l Anti-T₄-Fab'-Biotin aus polyklonalen Antikörpern gegen T4 (entsprechend Beispiel 1 hergestellt)
120 mmol/l Natriumbarbiturat
18,2 mmol/l Phosphatpuffer, pH 8,6
0,2 Gew.-% Rinderserumalbumin

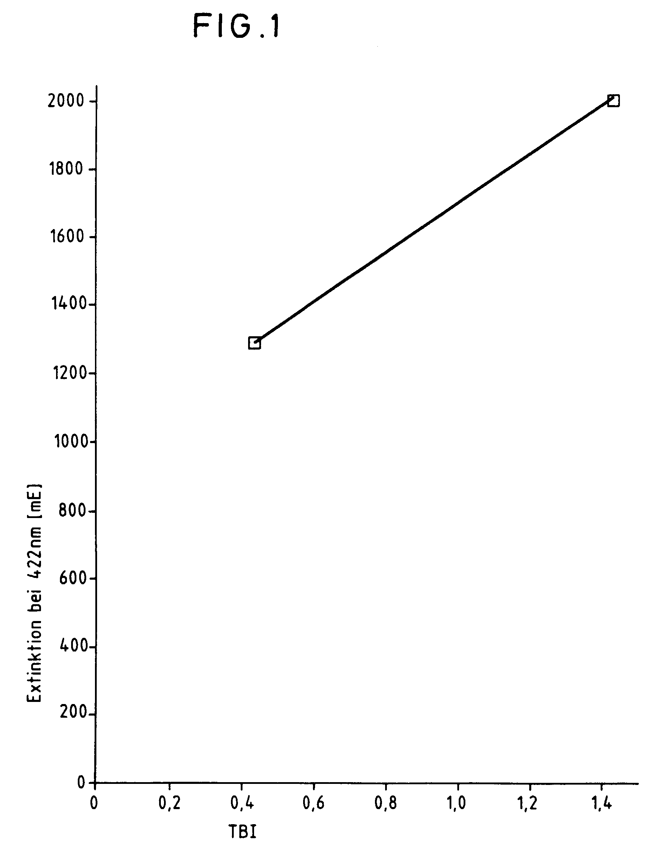

50 µl Probe werden mit 500 µl Reagenz 1 in ein mit Streptavidin beschichtetes Polystyrolgefäß gegeben und 30 Minuten bei 25°C inkubiert. Es werden 500 µl Reagenz 2 zugegeben und 30 Minuten bei 25°C inkubiert. Anschließend wird mit Wasser gewaschen und 1 ml ABTS®-Lösung (vgl. Beispiel 1) zugegeben, bei 25°C 30 Minuten inkubiert und die optische Dichte bei 422 nm als Maß für den Thyroxinbindungsindex bestimmt. Die Ergebnisse sind aus Fig. 1 zu ersehen.

### Beispiel 3

### Bestimmung eines Antikörpers gegen T4

Als Probe werden Standardlösungen von polyklonalen Antikörpern gegen T4, mit den Konzentrationen 0, 0,5 und 1,0 mg/l verwendet.

Reagenz:
10 mmol/l T4-Biotin-Konjugat
50 U/l Streptavidin-POD-Konjugat
120 mmol/l Nariumbarbiturat
18,2 mmol/l Phosphatpuffer, pH 8,6
1,27 mmol/l 8-Anilino-1-Naphthalinsulfonsäure
0,2 Gew.-% Rinderserumalbumin

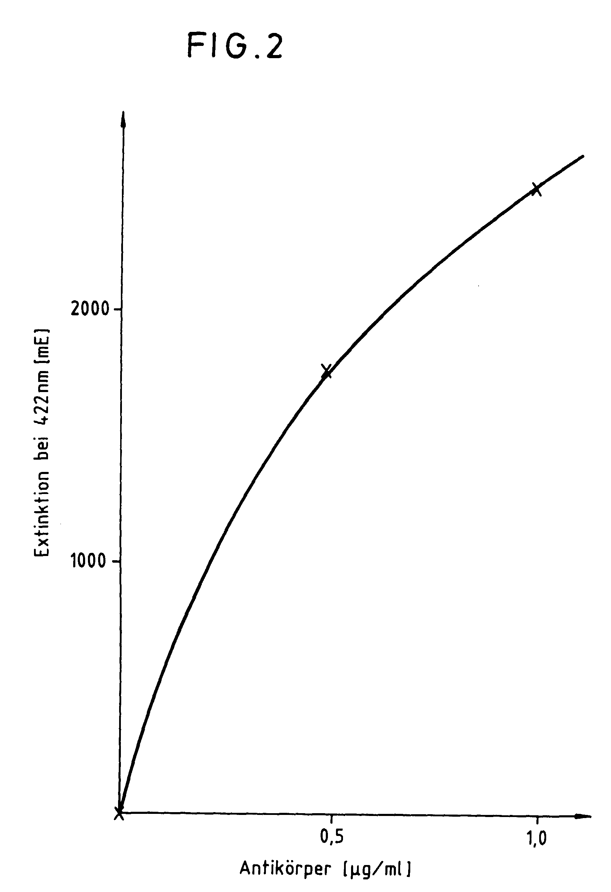

50 µl Probe und 1 ml Reagenz werden in einem mit Streptavidin beschichteten Polystyrolgefäß bei 25°C 30 Minuten inkubiert. Anschließend wird gewaschen und 1 ml ABTS®-Lösung (vgl Beispiel 1) zugegeben, bei 25°C 30 Minuten inkubiert und die optische Dichte bei 422 nm als Maß für den Gehalt an Antikörpern gegen T4 bestimmt. Die Ergebnisse sind aus Figur 2 zu ersehen.

### Beispiel 4

### Herstellung von IgG-Biotin (1:1)

1) 50 mg eines monoklonalen Antikörpers gegen TSH (ECACC 87122202) wird mit 2-fachem molarem Überschuß an D-Biotinyl-ξ-amidocapronsäure-N-hydroxy-succinimidester gem. JACS 100 (1978), 3585-3590 umgesetzt. Man erhält als Hauptprodukt monobiotinylierte IgG's und höherbiotinylierte Nebenprodukte, die abgetrennt werden.
2) Die Abtrennung erfolgt mittels DIP-Chromatographie (Delta-Isoelectric-Point) die z.B. in EP-A 0 131 343 beschrieben ist.

Die Trennung des Gemisches erfolgte auf einer Mono-S Kationenaustauschersäule (Pharmacia).

Das Gemisch wurde in 1 mmol/l Kaliumpyrophosphat-Puffer pH 6,9 (Puffer A) aufgegeben. Die gebundenen Komponenten wurden durch Anlegen eines linearen Gradienten mit 20 mmol/l Kaliumpyrophosphatpuffer/200 mmol/l NaCl, pH 6,9 (Puffer B) eluiert. Man erhält die reine monobiotinylyierte IgG Fraktion in hoher Ausbeute, neben Pools mit höherem Biotinylierungsgrad.

### Beispiel 5

### Herstellung von Fab-Biotin (1:1)

1) Monoklonale Antikörper gegen TSH (ECACC 87122202) werden gem. A. Johnstone, R. Thorpe; Immunochemistry in Practice, Blackwell Scientific Publications (1982), 52 -53, in Fab gespalten.
   50 mg Fab werden wie in Beispiel 4 beschrieben umgesetzt.
2) Die Auftrennung erfolgt hier unter modifizierten Bedingungen:
   Puffer A: 50 mmol/l MES pH 5,6
   Puffer B: 50 mmol/l MES/200 mmol/l NaCl pH 5,6
Unter diesen Bedingungen kann Fab-Biotin unter hohen Ausbeuten rein erhalten werden.

### Abkürzungen

### Biotin X-OSu:

N-Biotinoyl-ξ- Aminocapronsäure-hydroxysuccinimidester

### MES:

2- (N-Morpholino) ethansulfonsäure
KPP: Kaliumpyrophosphat
RSA: Rinderserumalbumin
POD: Peroxidase

### Beispiel 6

### TSH Test

Die Durchführung des Tests erfolgt mit einer Mischung aus den beiden monoklonalen Antikörper gegen TSH (ECACC 87122201 und ECACC 87122202) die gem. Beispiel 5 als Fab-Biotin hergestellt wurden.

### Schritt 1:

3 µg Fab-Biotin-Mischung in 1 ml 50 mmol/l Phosphatpuffer pH 7,5, 0,1 % RSA, werden mit 200 µl Probe (Humanserum aufgestockt mit TSH) für 2 Stunden in einem mit Streptavidin beschichteten Tube (Beispiel 1, siehe AFP) inkubiert. Danach wird ungebundenes Material ausgewaschen.

### Schritt 2:

200 mU Streptavidin-POD-Konjugat in lml Puffer (s.o.) wird 1 Stunde inkubiert und danach ungebundenes Material ausgewaschen.

### Schritt 3:

1 ml Substratlsg (Beispiel 1, siehe AFP) wird 1 Stunde inkubiert und die optische Dichte bei 422 nm als Maß für den TSH-Gehalt bestimmt.

## Patentansprüche

1. Verfahren zur Bestimmung eines Liganden,
**dadurch gekennzeichnet**,
daß man den zu bestimmenden Liganden mit
a) mindestens 2 Molekülen einer für P1 spezifischen bindefähigen Substanz P2, von denen mindestens eines dieser Moleküle eine Markierung trägt, und
b) einem Reagenz, umfassend Rezeptoren R, die den Liganden spezifisch binden, wobei jeder Rezeptor aus einem einzigen Molekül eines für P2 monovalent bindefähigen Bindungspartners P1 und dem einzig spezifischen Bindungsmolekül R₁ für den zu bestimmenden Liganden besteht und wobei P1 in allen Rezeptoren R gleich ist und R₁ in allen Rezeptoren gleich sein kann, umsetzt und dann die Markierung bestimmt.

2. Verfahren nach Anspruch 1,
**dadurch gekennzeichnet**,
daß man in heterogener Phase arbeitet und hierzu ein Molekül P2, das an eine feste Phase gebunden und nicht markiert ist, verwendet.

3. Verfahren nach Anspruch 1,
**dadurch gekennzeichnet**,
daß man in homogener Phase arbeitet und alle Moleküle P2 in markierter Form einsetzt.

4. Verfahren nach einem der vorhergehenden Ansprüche,
**dadurch gekennzeichnet**,
daß man zusätzlich ein Konjugat K aus Ligand oder Ligand-Analog und P1 bei der Inkubation zusetzt.

5. Verfahren nach einem der vorhergehenden Ansprüche,
**dadurch gekennzeichnet**,
daß P1 Biotin, ein Hapten oder ein Epitop ist.

6. Verfahren nach einem der vorhergehenden Ansprüche,
**dadurch gekennzeichnet**,
daß P2 Avidin, Streptavidin, ein Polymeres derselben oder ein Antikörper oder Antikörperfragment ist.

7. Verfahren nach einem der vorhergehenden Ansprüche,
**dadurch gekennzeichnet**,
daß R₁ ein Hapten, ein Epitop, eine DNA- oder RNA-Sequenz oder ein Makromolekül ist.

8. Reagenz zur Bestimmung eines Liganden,
**dadurch gekennzeichnet**,
daß es
a) mindestens 2 Moleküle einer für P1 spezifisch bindefähigen Substanz P2, wobei entweder mindestens eines der Moleküle P2 eine Markierung trägt und mindestens ein weiteres Molekül P2 an eine Festphase gebunden vorliegt oder alles P2 eine Markierung trägt und keine Festphase vorliegt, und
b) ein Reagenz, umfassend Rezeptoren R, die den Liganden spezifisch binden, wobei jeder Rezeptor aus einem einzigen Molekül eines für P2 monovalent bindefähigen Bindungspartners P1 und dem einzig spezifischen Bindungsmolekül R₁ für den zu bestimmenden Liganden besteht und wobei P1 in allen Rezeptoren R gleich ist und R₁ in allen Rezeptoren gleich sein kann, enthält.

9. Reagenz nach Anspruch 8,
**dadurch gekennzeichnet**,
daß es unmarkiertes, an eine Festphase gebundenes P2 enthält.

10. Reagenz nach Anspruch 8 oder 9,
**dadurch gekennzeichnet**,
daß es zusätzlich ein Konjugat K aus Ligand oder Ligand-Analog und P1 enthält.

## Claims

1. Method for the determination of a ligand,
**wherein**
the ligand to be determined is reacted with
a) at least 2 molecules of a specifically bindable substance P2 of which at least one of these molecules carries a label and
b) a reagent comprising receptors R that specifically bind the ligand wherein each receptor is composed of a single molecule of a binding partner P1 which is capable of monovalent binding to P2 and the single specific binding molecule R₁ for the ligand to be determined and wherein P1 in all receptors R is the same and R₁ in all receptors can be the same and then the label is determined.

2. Method as claimed in claim 1,
**wherein**
one works in a heterogeneous phase and for this a molecule P2 is used which is bound to a solid phase and is not labelled.

3. Method as claimed in claim 1,
**wherein**
one works in a homogeneous phase and all molecules P2 are employed in a labelled form.

4. Method as claimed in one of the previous claims,
**wherein**
in addition a conjugate C of a ligand or ligand analogue and P1 is added during the incubation.

5. Method as claimed in one of the previous claims,
**wherein**
P1 is biotin, a hapten or an epitope.

6. Method as claimed in one of the previous claims,
**wherein**
P2 is avidin, streptavidin, a polymer thereof or an antibody or antibody fragment.

7. Method as claimed in one of the previous claims,
**wherein**
R₁ is a hapten, an epitope, a DNA or RNA sequence or a macromolecule.

8. Reagent for the determination of a ligand,
**wherein**
it contains
a) at least 2 molecules of a specifically bindable substance P2 of which at least one of the molecules P2 carries a label and at least one further molecule P2 is present bound to a solid phase or all P2 carries a label and no solid phase is present and
b) a reagent comprising receptors R that specifically bind the ligand wherein each receptor is composed of a single molecule of a binding partner P1 which is capable of monovalent binding to P2 and the single specific binding molecule R₁ for the ligand to be determined and wherein P1 in all receptors R is the same and R₁ in all receptors can be the same.

9. Reagent as claimed in claim 8,
**wherein**
it contains unlabelled P2 bound to a solid phase.

10. Reagent as claimed in claim 8 or 9,
**wherein**
it additionally contains a conjugate C of ligand or ligand analogue and P1.

## Revendications

1. Procédé de détermination d'un ligand, caractérisé en ce que
l'on fait réagir le ligand à déterminer avec
a) au moins deux molécules d'une substance P2 capable de liaison spécifique pour P1, molécules parmi lesquelles l'une au moins porte un marqueur, et
b) un réactif comprenant des récepteurs R qui lient spécifiquement le ligand, chaque récepteur consistant en une seule molécule d'un partenaire de liaison P1 capable de liaison monovalente pour P2 et en la molécule de liaison à spécificité unique R₁ pour le ligand à déterminer, et P1 étant identique dans tous les récepteurs et R₁ pouvant être identique dans tous les récepteurs, puis on détermine le marqueur.

2. Procédé selon la revendication 1, caractérisé en ce que l'on opère en phase hétérogène et on utilise à cet effet une molécule P2 qui est liée à une phase solide et qui n'est pas marquée.

3. Procédé selon la revendication 1, caractérisé en ce que l'on opère en phase homogène et l'on utilise toutes les molécules P2 sous forme marquée.

4. Procédé selon l'une des revendications précédentes, caractérisé en ce que l'on ajoute en plus un conjugué K de ligand ou d'analogue de ligand et de P1 lors de l'incubation.

5. Procédé selon l'une des revendications précédentes, caractérisé en ce que P1 est la biotine, un haptène ou un épitope.

6. Procédé selon l'une des revendications précédentes, caractérisé en ce que P2 est l'avidine, la streptavidine, un polymère de celles-ci ou un anticorps ou fragment d'anticorps.

7. Procédé selon l'une des revendications précédentes, caractérisé en ce que R₁ est un haptène, un épitope, une séquence d'ADN ou d'ARN ou une macromolécule.

8. Réactif pour la détermination d'un ligand, caractérisé en ce qu'il contient
a) au moins deux molécules d'une substance P2 capable de liaison spécifique pour P1, au moins l'une des molécules P2 portant un marqueur et au moins une autre molécule P2 étant présente sous forme liée à une phase solide ou bien la totalité de P2 portant un marqueur et aucune phase solide n'étant présente, et
b) un réactif comprenant des récepteurs R qui lient spécifiquement le ligand, chaque récepteur consistant en une seule molécule d'un partenaire de liaison P1 capable de liaison monovalente pour P2 et en la molécule de liaison à spécificité unique R₁ pour le ligand à déterminer et P1 étant identique dans tous les récepteurs R et R₁ pouvant être identique dans tous les récepteurs.

9. Réactif selon la revendication 8, caractérisé en ce qu'il contient du P2 non marqué et lié à une phase solide.

10. Réactif selon la revendication 8 ou 9, caractérisé en ce qu'il contient en plus un conjugué K constitué par un ligand ou un analogue de ligand et de P1.
